**Europäisches Patentamt**

**European Patent Office**

**Office européen des brevets**

(19)

(11) Veröffentlichungsnummer: **0 268 864 B1**

(12) # EUROPÄISCHE PATENTSCHRIFT

(45) Veröffentlichungstag der Patentschrift: **05.02.92**

(21) Anmeldenummer: **87115956.2**

(22) Anmeldetag: **30.10.87**

(51) Int. Cl.⁵: **C07C 67/38**, C07C 69/533, B01J 31/20

(54) **Verfahren zur kontinuierlichen Herstellung von Pentensäurealkylestern.**

(30) Priorität: **08.11.86 DE 3638218**

(43) Veröffentlichungstag der Anmeldung:
**01.06.88 Patentblatt 88/22**

(45) Bekanntmachung des Hinweises auf die
Patenterteilung:
**05.02.92 Patentblatt 92/06**

(84) Benannte Vertragsstaaten:
**BE CH DE ES FR GB IT LI NL**

(56) Entgegenhaltungen:
**EP-A- 0 065 629**
**US-A- 3 976 670**

(73) Patentinhaber: **BASF Aktiengesellschaft**
**Carl-Bosch-Strasse 38**
**W-6700 Ludwigshafen(DE)**

(72) Erfinder: **Panitz, Paul**
**Wachenheimer Strasse 1**
**W-6520 Worms 1(DE)**
Erfinder: **Reitz, Heinrich, Dr.**
**Leibnizstrasse 3**
**W-6800 Mannheim 1(DE)**
Erfinder: **Schuch, Gunter, Dr.**
**Ulrich-v.-Hutten-Strasse 5**
**W-6700 Ludwigshafen(DE)**
Erfinder: **Seyfert, Wilfried, Dr.**
**Bachweg 8**
**W-6719 Weisenheim(DE)**

**Beschreibung**

Gegenstand der Erfindung ist ein Verfahren zur kontinuierlichen Herstellung von Pentensäurealkylestern durch Umsetzen von Butadien enthaltenden $C_4$-Schnitten mit Alkanolen und Kohlenmonoxid in Gegenwart von Kobaltcarbonylkatalysatoren und heterocyclischen aromatischen tertiären Stickstoffbasen bei einer Temperatur von 100 bis 160 °C und unter einem Druck von 400 bis 1200 bar.

Aus der DE-OS 34 13 448 ist ein Verfahren zur Herstellung von Pentensäurealkylestern bekannt, bei dem man Butadien enthaltende $C_4$-Schnitte mit Kohlenmonoxid und Alkanolen in Gegenwart von Kobaltcarbonylkatalysatoren und tertiären Stickstoffbasen umsetzt und das von überschüssigem Kohlenmonoxid befreite flüssige Reaktionsgemisch bei erhöhten Temperaturen unter einem Druck von 5 bis 80 bar mit Wasserstoff behandelt. Hierbei gelingt es zwar, bei genügend langer Einwirkung von Wasserstoff die verwendeten Kobaltcarbonylkatalysatoren in eine nicht flüchtige Form überzuführen, so daß bei der weiteren Aufarbeitung wesentliche Kobaltabscheidungen vermieden werden. Dieses Verfahren hat jedoch den Nachteil, daß die erzeugten Pentensäureester teilweise hydriert werden. Auch die in den restlichen Kohlenwasserstoffen enthaltenen Olefine werden teilweise hydriert und haben somit für eine weitere Verwendung einen verminderten Wert. Nach einem anderen in der DE-OS 28 02 580 beschriebenen Verfahren werden die bei der Herstellung von Pentensäurealkylestern aus Butadien enthaltenden $C_4$-Schnitten durch Carbalkoxylierung erhaltenen Reaktionsgemische nach Abtrennen der $C_4$-Kohlenwasserstoffe und des nichtumgesetzten Butadiens bei einer Temperatur von 100 bis 160 °C und einen Druck von 50 bis 200 bar mit Kohlenmonoxid behandelt. Das Verfahren hat den Nachteil, daß die abgetrennten Kohlenwasserstoffe erhebliche Mengen an flüchtigem Kobaltkatalysator enthalten, der sich bei der Destillation zersetzt. Die damit verbundenen Verstopfungen der Destillationskolonnen erfordern dann aufwendige Kolonnenspülungen, bei denen das Kobalt in einer inaktiven Form zurückgewonnen wird.

Es war deshalb die technische Aufgabe gestellt die Herstellung von Pentensäurealkylestern durch Carbalkoxilierung von Butadien enthaltenden $C_4$-Schnitten so zu gestalten, daß die Ausbeute an Pentensäurealkylestern nicht vermindert wird, der Kobaltkatalysator in eine nicht-flüchtige Form übergeführt wird, so daß bei der Aufarbeitung möglichst wenig Kobaltabscheidungen bei der Destillation eintreten.

Diese Aufgabe wird gelöst in einem kontinuierlichen Verfahren zur Herstellung von Pentensäurealkylestern durch Umsetzen von Butadien enthaltenden $C_4$-Schnitten mit Alkanolen und Kohlenmonoxid in Gegenwart von Kobaltcarbonylkatalysatoren und heterocyclischen aromatischen tertiären Stickstoffbasen bei einer Temperatur von 100 bis 160 °C und unter einem Druck von 100 bis 1200 bar, wobei man das so erhaltene Reaktionsgemisch in dem Maße wie es anfällt, bei einer Temperatur von 100 bis 160 °C unter einem Druck von 250 bis 1200 bar mit einer Verweilzeit von 5 bis 60 Minuten im wesentlichen ohne Rückvermischung durch eine Behandlungszone leitet.

Das neue Verfahren hat den Vorteil, daß der Aufarbeitung des Reaktionsgemisches die Abscheidung von Kobalt wesentlich vermindert wird. Ferner hat das neue Verfahren den Vorteil, daß die Ausbeute an Pentensäurealkylester nicht beeinträchtigt wird.

Erfindungsgemäß geht man von Butadien enthaltenden $C_4$-Schnitten aus. Als solche seien alle Gemische von überwiegend unverzweigten $C_4$-Kohlenwasserstoffen definiert, die mehr als 10 Gew.% Butadien und mehr als 15 Gew.% Butene enthalten. Je nach Provenienz liegen die einzelnen Komponenten in solchen Gemischen normalerweise in folgenden Anteilen vor

Butadien 10 bis 70, durchschnittlich 30 bis 60 Gew.%

Isobuten 15 bis 40, durchschnittlich 20 bis 35 Gew.%

But-1-en 10 bis 40, durchschnittlich 10 bis 25 Gew.%

But-2-en 5 bis 20, durchschnittlich 5 bis 15 Gew.%

Butane 1 bis 10, durchschnittlich 1 bis 10 Gew.%

Butine 0,1 bis 3, durchschnittlch 0,1 bis 3 Gew.%

Solche $C_4$-Schnitte fallen beispielsweise an bei der Dehydrierung von Butan oder Buten oder als Nebenprodukte bei der Ethylengewinnung durch thermische Spaltung (Cracken) von Flüssiggas (NLG), Leichtbenzin (Naphtha) oder höheren Kohlenwasserstoffschnitten.

Bevorzugt verwendet man $C_1$- $C_4$-Alkanole wie Methanol, Ethanol, Propanol, Butanol oder Isobutanol. Besonders bevorzugt wird Methanol verwendet. Vorteilhaft wendet man einen Überschuß an Alkanolen an, insbesondere 1, 1 bis 5 Mol je Mol Butadien.

Kohlenmonoxid wird vorteilhaft im Überschuß zum Beispiel dem 1,5- bis 10-fachen der stöchiometrisch erforderlichen Menge angewandt.

Die Umsetzung führt man bei einer Temperatur von 100 bis 160 °C, insbesondere 120 bis 145 °C durch. Hierbei hält man einen Druck von 100 bis 1200 bar, vorteilhaft von 400 bis 1200 bar, insbesondere von 500 bis 900 bar ein.

Die verwendeten Kobaltcarbonylkatalysatoren werden entweder in situ aus Kobaltsalzen, z.B. fettsauren Kobaltsalzen wie Formiat, Acetat, Propionat oder Butyrat erzeugt. Vorteilhaft bringt man den Katalysator bereits als Kobaltcarbonyl ein. Insbesondere hat es sich bewährt, wenn man den Kobaltcarbonylkatalysator gelöst im $C_4$-Schnitt in das Reaktionsgemisch einbringt. Eine solche Lösung erhält man beispielsweise durch Umsetzen einer wäßrigen Lösung von fettsauren Kobaltsalzen mit einem Gemisch aus Kohlenmonoxid und Wasserstoff in Gegenwart von Aktivkohle bei einer Temperatur von 100 bis 170°C und einem Druck von 100 bis 400 bar. Aus der wäßrigen Lösung wird das entstandene Kobaltcarbonyl dann mit Butadien enthaltendem $C_4$-Schnitt extrahiert. Vorteilhaft wird auch eine Teil des zurückgewonnenen Kobaltcarbonylkatalysators wieder für die Reaktion verwendet.

Je Mol Butadien verwendet man in der Regel 0,02 bis 0,2 Mol Kobaltcarbonylkatalysator.

Geeignete heterocyclische aromatische tertiäre Stickstoffbasen haben vorteilhaft einen pKa-Wert von 4 bis 9. Geeignete Stickstoffbasen sind beispielsweise Pyridin, 3-Methypyridin, 4-Methylpyridin, 2,3-Dimethylpyridin, 2,4-Dimethylpyridin, 3,5-Dimethylpyridin, 4-Benzylpyridin, Chinolin oder Isochinolin. Besonders bevorzugt werden Pyridin und 3- oder 4-Methylpyridin oder deren Gemische verwendet. Es hat sich bewährt, wenn man je Mol Butadien 0,5 bis 3 Mol der genannten Stickstoffbasen anwendet.

Die Reaktion kann in einer oder mehreren hintereinander geschalteten Reaktionszonen, z.B. 1 bis 4 Reaktionszonen durchgeführt werden. Das erhaltene Reaktionsgemisch enthält neben nichtumgesetztem Butadien andere $C_4$-Kohlenwasserstoffe aus dem $C_4$-Schnitt, heterocyclische aromatische tertiäre Stickstoffbasen, Kobaltcarbonylkatalysator, nicht umgesetzte Alkanole, die als Wertprodukte erzeugten Pentensäurealkylester sowie Nebenprodukte wie Valeriansäureester, Vinylcyclohexen sowie Polymerisate des Butadiens.

Nach dem erfindungsgemäßen Verfahren wird das so erhaltene Reaktionsgemisch in dem Maße wie es der ersten Stufe entnommen wird, im wesentlichen ohne Rückvermischung durch eine Behandlungszone als zweite Stufe geleitet. Diese Behandlungszone enthält vorteilhaft Einbauten wie Füllkörper oder Böden mit Düsenöffnungen, um Rückvermischung zu vermeiden. Die Behandlung erfolgt bei einer Temperatur von 100 bis 160, insbesondere 120 bis 145°C und unter einem Druck von 250 bis 1200 bar, insbesondere 500 bis 900 bar. Ferner hält man in der vorzugsweise rohrförmigen Behandlungszone eine Verweilzeit von 5 bis 60 Minuten, insbesondere 10 bis 30 Minuten ein. Zweckmäßig leitet man das Reaktionsgemisch aus der ersten Stufe ohne Abtrennung von Reaktionspartnern oder Reaktionsprodukten unter den in der ersten Stufe aufrechterhaltenen Druck- und Temperaturbedingungen durch die Behandlungszone. Es ist jedoch auch möglich, bei gegenüber der ersten Reaktionszone geänderten Temperaturen und/oder vermindertem Druck zu arbeiten.

Aus dem Austrag der zweiten Stufe wird Kohlenmonoxid abgetrennt und die flüssigen Anteile durch Destillation aufgearbeitet.

Die nach dem Verfahren der Erfindung erhältlichen Pentensäurealkylester eignen sich zur Herstellung von Adipinsäurealkylestern durch Carbalkoxilierung.

Das Verfahren sei an folgenden Beispielen veranschaulicht.

Beispiel

In eine Vorrichtung aus zwei hintereinander geschalteten Rührkesseln von 150 ml Inhalt werden dem ersten Kessel stündlich ein Gemisch aus 38 g $C_4$-Schnitt mit einem Gehalt an Butadien von 42 Gew.%, 10,3 g Methanol, 25 Nl Kohlenmonoxid, 3,6 g Dikobaltoctacarbonyl sowie 33 g Picolin zugeführt. Die Umsetzung wird in beiden Rührkesseln bei einer Temperatur von 135°C und unter einem Druck von 650 bar durchgeführt. Nach dem zweiten Rührkessel durchströmt das Reaktionsgemisch eine Behandlungszone mit einem Durchmesser von 1,7 cm und einer Länge von 16 cm. In der rohrförmigen Behandlungszone hält man eine Temperatur von 135°C und einen Druck von 650 bar aufrecht. Die Verweilzeit beträgt 15 Minuten. Das so erhaltene Reaktionsgemisch wird abgekühlt und entspannt. Es enthält lediglich 400 ppm flüchtiges Kobalt, von denen 200 ppm bei der destillativen Abtrennung der Reaktionsprodukte und Reaktanten als metallisches Kobalt abgeschieden werden.

Vergleichsbeispiel

Wiederholt man Beispiel 1 mit dem Unterschied, daß der Reaktionsaustrag nach der Carbalkoxilierung ohne weitere Behandlung direkt der destillativen Auftrennung zugeführt wird, so enthält der Reaktionsaustrag 2100 ppm Co in Form flüchtiger Verbindungen, von denen 1500 ppm in den Kolonnen abgeschieden werden.

**Patentansprüche**

1. Verfahren zur kontinuierlichen Herstellung von Pentensäurealkylestern durch Umsetzen von Butadien enthaltenden $C_4$-Schnitten mit Alkanolen und Kohlenmonoxid in Gegenwart von Kobaltcarbonylkatalysatoren und heterocyclischen aromatischen tertiären Stickstoffbasen bei einer Temperatur von 100 bis 160°C und unter einem Druck von 100 bis 1200 bar, dadurch gekennzeichnet, daß man das Reaktionsgemisch in dem maße wie es anfällt bei einer Temperatur von 100 bis 160°C unter einem Druck von 250 bis 1200 bar mit einer Verweilzeit von 5 bis 60 Minuten im wesentlichen ohne Rückvermischung durch eine Behandlungszone leitet.

2. Verfahren nach Anspruch 1, dadurch gekennzeichnet, daß man in einer rohrförmigen Behandlungszone eine Verweilzeit von 10 bis 30 Minuten einhält.

**Claims**

1. A process for the continuous preparation of an alkyl pentenoate by reacting a butadiene-containing $C_4$ cut with an alkanol and carbon monoxide in the presence of a cobalt carbonyl catalyst and a heterocyclic aromatic tertiary nitrogen base at from 100 to 160°C and under from 100 to 1,200 bar, wherein the reaction mixture is passed, at the rate at which it is obtained, through a treatment zone, at from 100 to 160°C under from 250 to 1,200 bar with a residence time of from 5 to 60 minutes, substantially without back-mixing.

2. A process as claimed in claim 1, wherein a residence time of from 10 to 30 minutes is maintained in a tubular treatment zone.

**Revendications**

1. Procédé de préparation continue d'esters alkyliques d'acide penténoïque par réaction de coupes en $C_4$ contenant du butadiène avec des alcanols et du monoxyde de carbone en présence de catalyseurs au cobalt-carbonyle et de bases azotées tertiaires aromatiques hétérocycliques, à une température de 100 à 160°C et sous une pression de 100 à 1200 bar, caractérisé en ce qu'on fait passer le mélange réactionnel à mesure qu'il se forme, dans une zone de traitement à une température de 100 a 160°C sous une pression de 250 à 1200 bar avec un temps de séjour de 5 à 60 minutes substantiellement sans remélangeage.

2. Procédé selon la revendication 1, caractérisé en ce qu'on maintient dans une zone de traitement tubulaire un temps de séjour de 10 à 30 minutes.